# EUROPEAN PATENT SPECIFICATION

(11) **EP 3 625 802 B1**
(45) Date of publication and mention of the grant of the patent: **23.08.2023**
(21) Application number: 18746109.0
(22) Date of filing: 09.07.2018
(51) Int. Cl.: G16H 15/00, G16H 50/20, G16H 10/40, G16B 30/00, G16B 30/10, G16B 99/00

(54) **SCANSOFT : A METHOD FOR THE DETECTION OF GENOMIC DELETIONS AND DUPLICATIONS IN MASSIVE PARALLEL SEQUENCING DATA**
SCANSOFT: VERFAHREN ZUR DETEKTION VON GENOMISCHEN DELETIONEN UND DUPLIKATIONEN IN MASSIVEN PARALLELEN SEQUENZIERUNGSDATEN
SCANSOFT : PROCÉDÉ DE DÉTECTION DE SUPPRESSIONS GÉNOMIQUES ET DE DUPLICATIONS EN DONNÉES DE SÉQUENÇAGE PARALLÈLE MASSIF

(30) Priority: 20.07.2017 EP 17182324
(43) Date of publication of application: 25.03.2020
(73) Proprietor: Siemens Healthcare GmbH, 91052 Erlangen (DE)
(72) Inventor: HEUCKMANN, Johannes, 50169 Kerpen (DE); MARIOTTI, Erika, 50858 Köln (DE); ZACHERLE, Tobias, 50668 Köln (DE)
(86) International application number: PCT/EP2018/068523
(87) International publication number: WO 2019/016009

(56) References cited:
- WO-A1-2015/100427
- WO-A2-2010/028288
- US-A1- 2015 142 328
- ZHONGWU LAI ET AL: "VarDict: a novel and versatile variant caller for next-generation sequencing in cancer research", NUCLEIC ACIDS RESEARCH, vol. 44, no. 11, 7 April 2016 (2016-04-07) , pages e108-e108, XP055500343, ISSN: 0305-1048, DOI: 10.1093/nar/gkw227
- LI HENG ET AL: "MAPPING SHORT DNA SEQUENCING READS AND CALLING VARIANTS USING MAPPING QUALITY SCORES", GENOME RESEARCH, COLD SPRING HARBOR LABORATORY PRESS, US, vol. 18, 1 January 2008 (2008-01-01), pages 1851-1858, XP001503357, ISSN: 1088-9051, DOI: 10.1101/GR.078212.108 [retrieved on 2008-08-19]
- ERIC J DUNCAVAGE ET AL: "Hybrid capture and next-generation sequencing idetnify viral integration sites from formalin-fixed paraffin-embedded tissue", JOURNAL OF MOLECULAR DIAGNOSTICS,THE, AMERICAN SOCIETY FOR INVESTIGATIVE PATHOLOGY, US , vol. 13, no. 3 1 May 2011 (2011-05-01), pages 325-333, XP002683089, ISSN: 1525-1578, DOI: 10.1016/J.JMOLDX.2011.01.006 Retrieved from the Internet: URL:http://www.sciencedirect.com/science/a rticle/pii/S1525157811000249 [retrieved on 2018-01-10]
- RENDONG YANG ET AL: "ScanIndel: a hybrid framework for indel detection via gapped alignment, split reads and de novo assembly", GENOME MEDICINE, vol. 7, no. 1, 1 December 2015 (2015-12-01), XP055500309, DOI: 10.1186/s13073-015-0251-2

## Description

### TECHNICAL FIELD

The present invention relates to a method of identifying structural genomic rearrangements in massively parallel nucleic acid sequencing data, as well as an in vitro method to detect genomic alterations for stratifying patients for cancer therapy, including a step of identifying structural genomic rearrangements. Also provided is a method of generating a report including information on the identified rearrangement.

### BACKGROUND

Cancer genomes can harbor a broad spectrum of genomic alterations. The most frequently observed alterations include point mutations, small insertions and deletions, copy number alterations and gene fusions/translocations. In certain cases, additional complex alterations such as large deletions as well as duplications of defined genomic regions have been observed. The length of the altered i.e. deleted or duplicated DNA sequence can vary and may range from one or a few nucleotides to hundreds of thousands of bases. Several small insertions and deletions in the genome, such as, for example, EGFR Exon 19 deletions in Non-Small Cell Lung Cancer, are already tested on a routine basis. Large deletions or duplications such as, for example, N- and C-terminal deletions, as well as kinase duplications in EGFR are more difficult to detect and may require the performance of massive parallel or next-generation sequencing (NGS) approaches. NGS approaches typically provide a huge amount of relatively short sequence reads, which are either generated by single or paired-end sequencing.

Different approaches for the detection of structural variants (SV) such as genomic duplications and deletions of DNA segments through NGS approaches have been proposed. Typically, duplications and deletions can be detected by exploiting the orientation and the insert size of read pairs. For example, a region containing a genomic rearrangement may be detected by the identification of clusters of discordant reads pairs. The orientation of the read pairs allows classifying the type of rearrangement as duplication or deletion. False positive rearrangements can be filtered out by using confidence scores, the size of anchoring regions and the coverage of the genome. Examples of corresponding algorithms include BreakDancer as described in Chen et al., Nature Methods, 2009; and CLEVER as described in Marschall et al., Bioinformatics, 2012.

A further development of this approach is the algorithm FACTERA (Newman et al., Bioinformatics, 2014), which considers the reads spanning DNA double strand break points associated with the rearrangement. The orientation and the sequence of detected spanning reads accordingly yield additional and useful information on the type and identity of a genomic rearrangement.

In alternative settings, duplications and deletions can be identified by detecting significant variations in the number of reads covering a certain genomic region. This approach makes use of the fact that under the assumption of a homogenous coverage distribution of the whole genome, a significantly smaller or larger number of reads aligns to regions of the reference genome, which are deleted or duplicated. However, in order to account for variations of read depth due to experimental biases etc. additional segmentation algorithms must be employed. The method is thus only suitable for the detection of deletions and duplications, which are significantly larger than 100 bps. Additionally, this approach does not allow determining the exact breakpoint.

A different method currently in use for SV detection is based on the assembly of sequence reads without the use of a reference genome, i.e. a de novo assembly. Reads with sufficient amount of overlapping parts at the start or the end positions are used to form contigs, i.e. sets of mutually overlapping reads. Examples of corresponding algorithms include Cortex (Iqbal et al., Nature Genetics, 2012) and SPAdes (Bankevich et al., Journal of Computational Biology, 2012) .

A further possibility to detect SVs is provided by the recognition of read pairs for which one read is uniquely mapped to the reference genome whereas the other read is unmapped, i.e. so called split reads. The underlying assumption is that one read is unmapped because it is spanning a double strand breakpoint due to a genomic rearrangement. The unmapped read is considered to determine the breakpoint. Typically, the sequence of the unmapped read is split in segments of different length, which are mapped to the different positions on the reference genome (Ye et al., Bioinformatics, 2009; Karakoc et al., Nature Methods, 2012).

In another approach, an algorithm was developed which does not rely on the detection of discordant reads, but only on the identification of reads spanning the break point. This process allows identifying the two genomic regions involved in the rearrangement, which are successively scanned (CREST; J. Wang et al., Nature Methods, 2011).

ZHONGWU LAI ET AL: "VarDict: a novel and versatile variant caller for next-generation sequencing in cancer research",NUCLEIC ACIDS RESEARCH, describes a method for identifying structural genomic rearrangements according to the preamble of claim 1 and represents the closest prior art.

Thus, the currently used approaches are limited by constraints as to the size of the detectable structural genomic rearrangements, are based on burdensome and time consuming assembly schemes, typically requiring external tools, or do not provide information on the exact break point. There is hence a need for an improved methodology allowing detecting duplications, deletions and inversions in massively parallel nucleic acid sequencing data.

### SUMMARY

The present invention addresses this need and presents a method of identifying structural genomic rearrangements in massively parallel nucleic acid sequencing data, comprising:
(a) obtaining massively parallel sequencing information for one or more genomic regions as nucleic acid sequence reads;
(b) aligning said nucleic acid sequencing reads to one or more reference sequences; (c) selecting nucleic acid sequencing reads which only partially map to said reference sequence, wherein a portion of the nucleic acid sequencing reads remains unmapped, constituting a soft-clipped region;
(d) creating groups of nucleic acid sequencing reads as selected in step (c), all of which are defined by identical start or end positions of said soft-clipped regions; (e) generating a synthetic consensus sequence for each group as obtained in step (d); (f) generating reasonable combinations of positions between groups of nucleic acid sequencing reads, wherein soft-clipped nucleotides are at the start of the nucleic acid sequence, and groups of nucleic acid sequencing reads, wherein said soft-clipped nucleotides are at the end of the nucleic acid sequence by comparing the synthetic consensus sequence of step (e) with the reference sequence;
(g) pairing nucleic acid sequencing reads which match at respective positions in the reference sequence; and (h) detecting a structural genomic rearrangement if both synthetic consensus sequences of pairs as obtained in step (g) match at respective positions in the reference sequence.

The provided method thus advantageously uses soft-clipped reads, i.e. reads for which a number of nucleotides was clipped, meaning ignored, by the aligner in order to map the rest of the sequence of the read to the reference sequence, to identify break points of structural genomic rearrangements in a broad length range of about 10 bp to more than 10,000 bp. By allowing for the use of noise filtering steps and by a suitable sequence comparison approach, excluding the use of discordant read pairs, an efficient identification of structural genomic rearrangements becomes possible.

In one embodiment, the rearrangement is a deletion, a duplication or an inversion.

In a further embodiment of the present invention, the soft-clipped nucleotides of the nucleic sequencing read is at least 8 to 15 nucleotides long.

According to another embodiment of the present invention, alignment operations and sequence comparisons are performed with a string matching algorithm.

In a further embodiment, the massively parallel sequence information is provided in a format providing information on alignment and soft-clipped regions. Preferred formats are the BAM, SAM or CRAM format.

In yet another embodiment of the present invention, the nucleic acid sequencing reads have a length of about 50 nucleotides to 50 kb.

Further envisaged is that in the soft-clipped sequencing reads obtained in step (c) of the method as defined above, information on the position of mapped portion of said reads is stored electronically.

In a further embodiment, in step (d) of the method as defined above groups are discarded which comprise less than a predefined number of members. It is preferred that the number of members is 1, 2, 3, 4, 5, 6, 7 or 8.

In another embodiment of the present invention, the synthetic consensus sequence is identical to the corresponding sequence of a predefined number of sequencing reads in the group of nucleic acid sequencing reads as defined in step (d) of the method as defined above. It is preferred that said predefined number of sequencing reads is 1, 2, 3, 4 or more.

In a further embodiment of the method as defined above, in step (f) combinations of nucleic acid sequencing reads are discarded from further analysis, which are characterized by repetitive consensus sequences and/or which show a distance between the soft-clipped positions of the nucleic acid sequencing reads with respect to the reference sequence of more than 100 kb, preferably more than 35 kb.

In yet another embodiment of the present invention, the method comprises an additional step of elucidating the sequencing depth at the position of the detected structural genomic rearrangement and/or the position of the detected structural genomic rearrangement with respect to annotated functional information, preferably the gene name, or the location in intron, exon, promoter, enhancer, telomeric, pseudogenic, repetitive regions.

The present invention further envisages embodiments, wherein combinations of positions between groups of nucleic acid sequencing reads as obtained in step (f) of a method as defined herein above are considered to represent: (i) a duplication, if the ending position with respect to the reference sequence of the soft-clipped regions of said groups of nucleic acid sequencing reads which have a partially aligning portion at the start of the mapped nucleic acid sequence is smaller than the starting position with respect to the reference sequence of the soft-clipped regions of said groups of nucleic acid sequencing reads which have a soft-clipped region at the end of the mapped nucleic acid sequence read; (ii) a deletion, if the ending position with respect to the reference sequence of the soft-clipped regions of said groups of nucleic acid sequencing reads which have a partially aligning portion at the start of the mapped nucleic acid sequence is larger than the starting position with respect to the reference sequence of the soft-clipped regions of said groups of nucleic acid sequencing reads which have a soft-clipped region at the end of the mapped nucleic acid sequence; or (iii) an inversion, if pairs of said groups of nucleic acid sequencing reads which have a soft-clipped region can be formed, for which both members of the pair have a soft-clipped region at the start of the mapped nucleic acid sequence, or if both members of the pair have a soft-clipped region at the end of the mapped nucleic acid sequence.

In a further aspect, the present invention relates to an in vitro method to detect genomic alterations for stratifying patients for cancer therapy, comprising: (a) performing a massively parallel nucleic acid sequencing of nucleic acids extracted from a patient tumor sample; (b) identifying a structural genomic rearrangement; and (c) attributing the detection of a structural genomic rearrangement to the presence of genomic alterations which can guide a treatment decision.

In an embodiment said method additionally comprises a preparation step for nucleic acids extracted from a patient sample, which precedes step (a), comprising a hybrid-capture based nucleic acid enrichment for genomic regions of interest.

In another embodiment of the method, said genomic region of interest is a gene or region known to be relevant in cancer.

It is further preferred that the sample as mentioned above comprises one or more premalignant or malignant cells; cells from a solid tumor or soft tissue tumor or a metastatic lesion; tissue or cells from a surgical margin; a histologically normal tissue obtained in a biopsy; one or more circulating tumor cells (CTC); a normal, adjacent tissue (NAT) from a subject having a tumor or being at risk of having a tumor; or a blood, plasma or serum sample from the same subject having a tumor or being at risk of having a tumor; or is a corresponding paraffin or FFPE-sample.

In a further embodiment, the cancer may be breast cancer, prostate cancer, ovarian cancer, renal cancer, lung cancer, pancreas cancer, urinary bladder cancer, uterus cancer, kidney cancer, brain cancer, stomach cancer, colon cancer, melanoma or fibrosarcoma, gastrointestinal stromal tumor (GIST), glioblastoma or hematological leukemia or a lymphoma, both from the myeloid and lymphatic lineage.

In another embodiment, the method to detect genomic alterations for stratifying patients of the present invention further comprises providing a report in electronic, web-based, or paper form, to a patient or to another person or entity, a caregiver, a physician, an oncologist, a hospital, clinic, third party payor, insurance company or government office.

It is preferred that the report comprises one or more of: (i) output from the method, comprising the identification of the structural genomic rearrangement or wild-type sequence associated with a tumor of the type of the sample; (ii) information on the role of a genomic alteration, or corresponding wild-type sequence, in a disease, wherein said information comprises information on prognosis, resistance, or potential or suggested therapeutic options; (iii) information on the likely effectiveness of a therapeutic option, the acceptability of a therapeutic option, or the advisability of applying the therapeutic option to a patient having a structural genomic rearrangement identified in the report; (iv) information, or a recommendation on the administration of a drug, the administration at a preselected dosage, or in a preselected treatment regimen, in combination with other drugs, to the patient; or wherein (v) not all structural genomic rearrangements identified in the method are specified in the report, the report can be limited to alterations in genes of clinical relevance.

### BRIEF DESCRIPTION OF THE DRAWINGS

FIG. 1 shows a schematic illustration of soft-clipped sequencing reads representing a duplication-type structural genomic rearrangement.
FIG.2 schematically depicts soft-clipped reads mapped to one reference genome in a duplication-type structural genomic rearrangement.
FIG. 3 shows the start and end positions of soft-clipped regions in a duplication-type structural genomic rearrangement.
FIG. 4 depicts the duplicated sequence, which has been identified in accordance with the mapping shown in Fig. 4.
FIG. 5 depicts a situation in which soft-clipped sequences of one genomic breakpoint map to a reference genome at another genomic breakpoint, which is characteristic for a duplication-type structural genomic rearrangement.
FIG. 6 depicts the same situation as in Fig. 5 in which soft-clipped sequences of one genomic breakpoint map to a reference genome at another genomic breakpoint, which is characteristic for a duplication-type structural genomic rearrangement. Here, the second breakpoint of the duplication is shown. Only if both breakpoints (i.e. the one shown in Fig. 5 and the one shown in Fig. 6) are present, a bona fide duplication has been detected.
FIG. 7 shows a schematic illustration of soft-clipped sequencing reads representing a deletion-type structural genomic rearrangement.
FIG. 8 shows the start and end positions of soft-clipped regions in a deletion-type structural genomic rearrangement.
FIG. 9 shows soft-clipped regions, which are not mapped to a reference sequence, indicating a deletion-type structural genomic rearrangement.
FIG. 10 depicts a situation in which soft-clipped sequences of one genomic breakpoint map to a reference at another genomic breakpoint, which is characteristic for a deletion-type structural genomic rearrangement.
FIG. 11 depicts the same situation as in Fig. 10, i.e. a situation in which soft-clipped sequences of one genomic breakpoint map to a reference at another genomic breakpoint, which is characteristic for a deletion-type structural genomic rearrangement. Only if both breakpoints (i.e. the one shown in Fig. 10 and the one shown in Fig. 11) are present, a bona fide deletion has been detected.

### DETAILED DESCRIPTION OF EMBODIMENTS

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" or "essentially consisting of" is considered to be a preferred embodiment of the term "comprising of". If hereinafter a group is defined to comprise at least a certain number of embodiments, this is meant to also encompass a group which preferably consists of these embodiments only.

Furthermore, the terms "(i)", "(ii)", " (iii)" or "(a)", "(b)", "(c)", "(d)", or "first", "second", "third" etc. and the like in the description or in the claims, are used for distinguishing between similar elements and not necessarily for describing a sequential or chronological order.

It is to be understood that the terms so used are interchangeable under appropriate circumstances and that the embodiments of the invention described herein are capable of operation in other sequences than described or illustrated herein. In case the terms relate to steps of a method, procedure or use there is no time or time interval coherence between the steps, i.e. the steps may be carried out simultaneously or there may be time intervals of seconds, minutes, hours, days, weeks etc. between such steps, unless otherwise indicated.

It is to be understood that this invention is not limited to the particular methodology, protocols etc. described herein as these may vary.

The drawings are to be regarded as being schematic representations and elements illustrated in the drawings are not necessarily shown to scale. Rather, the various elements are represented such that their function and general purpose become apparent to a person skilled in the art.

Unless defined otherwise, all technical and scientific terms used herein have the same meanings as commonly understood by one of ordinary skill in the art.

As has been set out above, the present invention concerns in one aspect a method of identifying structural genomic rearrangements in massively parallel nucleic acid sequencing data, comprising: (a) obtaining massively parallel sequencing information for one or more genomic regions as nucleic acid sequence reads; (b) aligning said nucleic acid sequencing reads to one or more reference sequences; (c) selecting nucleic acid sequencing reads which only partially map to said reference sequence, wherein a portion of the nucleic acid sequencing reads remains unmapped, constituting a soft-clipped region; (d) creating groups of nucleic acid sequencing reads as selected in step (c), all of which are defined by identical start or end positions of said soft-clipped regions; (e) generating a synthetic consensus sequence for each group as obtained in step (d); (f) generating reasonable combinations of positions between groups of nucleic acid sequencing reads, wherein soft-clipped nucleotides are at the start of the nucleic acid sequence, and groups of nucleic acid sequencing reads, wherein said soft-clipped nucleotides are at the end of the nucleic acid sequence by comparing the synthetic consensus sequence of step (e) with the reference sequence; (g) pairing nucleic acid sequencing reads which match at respective positions in the reference sequence; and (h) detecting a structural genomic rearrangement if both synthetic consensus sequences of pairs as obtained in step (g) match at respective positions in the reference sequence.

As used herein, a "structural genomic rearrangement" relates to an alteration of a genomic sequence in comparison to a reference sequence, which does not include single or small fragment nucleotide modifications or polymorphisms, e.g. up to about a length of about 5 nucleotides, such as nucleotide insertions, deletions or changes, as well as copy number alterations or gene fusions or translocations. The term in particular relates to alterations of sequence stretches of at least 5 nucleotides up to several kb. In preferred embodiments, structural genomic rearrangements according to the present invention are duplications, deletions or inversions.

The term "massively parallel nucleic acid sequencing data" as used herein relates to sequence data obtained by any technique suitable to provide sequence data in a high-throughput approach. It typically includes next-generation sequence (NGS) or second generation sequencing techniques.

The massively parallel sequencing approach includes any sequencing method that determines the nucleotide sequence of either individual nucleic acid molecules or expanded clones for individual nucleic acid molecules in a highly parallel fashion. For example, more than 10⁵ molecules may be sequenced simultaneously. The sequencing may be performed according to any suitable massive parallel approach. Typical platforms include Roche 454, GS FLX Titanium, Illumina, Life Technologies Ion Proton, Solexa, Solid or Helicos Biosciences Heliscope systems.

Obtaining massively parallel sequencing information means that any suitable massively parallel sequencing approach as mentioned, or as known to a skilled person, can be performed. The sequencing may include the preparation of templates, the sequencing, as well as subsequent imaging and initial data analysis steps.

Preparation steps may, for example, include randomly breaking nucleic acids such as genomic DNA, into smaller sizes and generating sequencing templates such as fragment templates. Spatially separated templates can, for example, be attached or immobilized at solid surfaces which allows for a sequencing reaction to be performed simultaneously. In typical examples, a library of nucleic acid fragments is generated and adaptors containing universal priming sites are ligated to the end of the fragments. Subsequently, the fragments are denatured into single strands and captured by beads. After amplification and a possible enrichment, e.g. as defined in more details herein below, a huge number of templates may be attached or immobilized in a polyacrylamide gel, or be chemically crosslinked to an amino-coated glass surface, or be deposited on individual titer plates. Alternatively, solid phase amplification may be employed. In this approach forward and reverse primers are typically attached to a solid support. The surface density of amplified fragments is defined by the ratio of the primers to the template on the support. This method may produce millions of spatially separated template clusters which can be hybridized to universal sequencing primers for massively parallel sequencing reactions. Further suitable options include multiple displacement amplification methods.

Suitable sequencing methods include, but are not limited to, cyclic reversible termination (CRT) or sequencing by synthesis (SBS) by Illumina, sequencing by ligation (SBL), single-molecule addition (pyrosequencing) or real-time sequencing. Exemplary platforms using CRT methods are Illumina/Solexa and HelicoScope. Exemplary SBL platforms include the Life/APG/SOLiD support oligonucleotide ligation detection. An exemplary pyrosequencing platform is Roche/454. Exemplary real-time sequencing platforms include the Pacific Biosciences platform and the Life/Visi-Gen platform. Other sequencing methods to obtain massively parallel nucleic acid sequence data include nanopore sequencing, sequencing by hybridization, nano-transistor array based sequencing, scanning tunneling microscopy (STM) based sequencing, or nanowire-molecule sensor based sequencing. Further details with respect to the sequencing approach would be known to the skilled person, or can be derived from suitable literature sources such as Goodwin et al., Nature Reviews Genetics, 2016, 17, 333-351, or van Dijk et al., Trends in Genetics, 2014, 9, 418-426.

A preferred sequencing method is sequencing by synthesis.

Correspondingly obtained data are provided in the form of sequencing reads. In a preferred embodiment, the sequencing read is a pair-end read. Obtaining such sequencing data may further include the addition of assessment steps or data analysis steps. For example, the sequencing reads may already have been aligned to a reference genome.

Furthermore, the presently described methodology may be used with any suitable sequencing read length. It is preferred to make use of sequencing reads of a length of about 50 to about 150 nucleotides, e.g. 50, 60, 70, 80, 90, 100, 110, 120, 130, 140, 150 or more nucleotides or any value in between the mentioned values. Most preferably, a length of 80 nucleotides is employed.

The terms "alignment" or "sequence alignment" or "aligning" as used herein relate to the process of sequence comparison and matching a sequencing read with a sequence location, e.g., a genomic location. In the context of the present invention alignment exclusively relates to nucleotide sequences. Aligned sequences of nucleotides are typically represented as rows within a matrix. Gaps are inserted between the residues so that identical or similar characters are aligned in successive columns. For the performance of an alignment operation or sequence comparison any suitable algorithm or tool can be used. For example, the present invention envisages the use of any string matching algorithm known to the skilled person. Preferred is an algorithm such as the Burrows-Wheeler Aligner (BWA), e.g. as described by Li and Durbin, 2009, Bioinformatics, 25, 1754-1760.

Information on the position where an alignment correspondence between a sequencing read and a reference sequence was detected may be stored together with the sequence information. For example, position information, information on the degree of correspondence, version and identity information on the reference sequence etc. may be stored together with the sequence information. In preferred embodiments, a format such as BAM, SAM or CRAM may be used. BAM and SAM formats are designed to contain the same information. The SAM format is a human readable format, and easier to process by conventional text based processing programs, such as, for example, standard Linux commands or python. The BAM format provides binary versions of the same data, and is designed to provide a good compression rate. The CRAM format is similar to the BAM format. In this format the compression is driven by the reference the sequence data is aligned to.

The term "reference sequence" as used herein relates to a sequence, which is used for alignment purposes within the context of the present invention. The reference sequence is typically a genomic sequence or part of a genomic sequence. The sequence may either be provided in a sense direction, or in a reverse-complement direction. This may depend on the type of structural genomic rearrangement to be detected. For deletions and duplications, comparison to sense reference sequences are preferred. The term "sense" or "sense orientation" corresponds to the plus strand of a duplex nucleic acid. The detection of inversions typically requires matching with respect to the reverse-complementary reference sequence. The term "reverse complementary", "reverse-complement" or "reverse complementary orientation" corresponds to the minus strand of a duplex nucleic acid. The reference sequence may be selected as any suitable genomic sequence derivable from databases as known the skilled person. For example, a reference sequence may be derived from the reference assembly provided by the Human Genome Reference Consortium. Also envisaged are further similar reference sequences. In specific embodiments, the reference sequence may include, but is not limited to, non-human genomic sequences such as monkey-, mouse-, rat-, bovine-sequences etc. The reference sequence may further be limited to certain sectors of the genome, e.g. specific chromosomes, or parts of a chromosome, or certain genes, groups of genes or gene clusters etc. Particularly preferred are sectors, which correspond to known mutational hotspots or which have been described as being involved in the etiology of diseases, in particular of cancer. In further embodiments, the reference sequence may be a sequence which has initially been obtained from a database as described above and which has been modified or corrected in accordance with sequencing reads analyzed in the context of the present invention, e.g. as mentioned herein above or below. For example, in case sequencing reads, preferably more than 3 sequencing reads, show consistently identical stretches of nucleotides or identical nucleotides in non-soft-clipped portions, which are, however, not present in the initial, database-derived sequence, such stretches of nucleotides or nucleotides may be introduced in the reference sequence and replace there the initially present information. Alternatively, the reference sequence may be a de novo sequence, which has, for example, been generated on the basis of sequencing reads as analyzed in the context of the present invention or described herein. Such a de novo sequence may further be compared or fused with a database-derived sequence. In a further, alternative embodiment, the reference sequence may correspond to a consensus sequence obtained from non-soft-clipped sequencing reads as analyzed in the context of the present invention. The wording obtaining massively parallel sequencing information "for one or more genomic regions" as used herein accordingly relates to the acquirement of sequence information as described above for either the entire genome of a subject, or for a subset thereof. Such a sub-set may be a chromosome, more than one chromosome, or a sub-chromosomal region. Such regions may further comprise more than one sub-chromosomal region from two or more chromosomes. In certain embodiments, the genomic regions may comprise stretches of 1 to 500 genes, e.g. stretches of 1 to 10, 1 to 20, 1 to 30, 1 to 40, 1 to 50, 1 to 60, 1 to 70, 1 to 80, 1 to 90, 1 to 100, 1 to 150, 1 to 200, 1 to 250, 1 to 300, 1 to 350, 1 to 400, 1 to 450 genes or stretches of any number of genes between the mentioned values, non-coding regions between genes, mutational hotspots which have been described in the literature or are known to the skilled person show mutations in a higher frequency etc. Preferably, a genomic region may have a size of between about 1 to 15 Mb, e.g. 15 Mb, 10 Mb, 7 Mb, 5 Mb, 3 Mb, 2 Mb, 1.5 Mb, 1 Mb, or 900 kb, 800 kb, 700 kb, 600 kb, 500 kb, 400 kb, 300 kb, 200 kb, 150 kb, 140 kb, 130 kb, 120 kb, 110 kb, 100 kb, 90 kb, 80 kb, 70 kb, 60 kb, 50 kb, 40 kb, 30 kb, 20 kb, or 10 kb or any size between the mentioned values.

In a central embodiment of the present invention sequencing reads, which have been aligned to a reference sequence as defined above, are selected if they show only a partial mapping or partial correspondence to the reference sequence. Typically, in such a situation, the sequence reads would be disregarded from further analysis, e.g. due to assumed sequence errors. Such partially mapping sequencing reads can advantageously be used to effectively detect structural genomic rearrangements such as duplications, deletions or inversions. In said partially mapping sequencing reads a portion of the nucleic acid sequencing read thus remains unmapped. In this scenario, in which thus only a certain percentage or number of the nucleotides shows a perfect correspondence with, or can perfectly be mapped to, a reference sequence, the sequencing reads are treated such that the remaining, i.e. non-mapping, nucleotides are marked to be masked or ignored in the corresponding data file, e.g. the BAM; SAM or CRAM file. These remaining unmapped nucleotides, which may occur at the end or the start of the sequencing read, i.e. the 5' or 3' terminus, are thus "soft-clipped". The term "soft-clipped" nucleotides thus relates to nucleotides in the direction 5' to 3' of a sequencing read which are not part of an alignment, but which have not been removed from the sequencing read, e.g. in a SAM or BAM file. Typically such soft-clipped nucleotides are not used by variant callers. In addition, soft-clipped sequences may not be used in calculation procedures for coverage. However, since soft-clipped sequencing reads may conceal a structural variation of a genomic sequence, the currently envisaged method comprises a specific selection step for this type of sequencing read. The number of unmapped, i.e. soft-clipped nucleotides per sequencing read may be at least 8. More preferably, the number of unmapped, i.e. soft-clipped nucleotides per sequencing read may be at least 9, 10, 11, 12, 13, 14 or 15. Particularly preferred is a number of at least 15 unmapped, i.e. soft-clipped nucleotides. Higher numbers such as 16, 17, 18, 19, 20 and more nucleotides are also envisaged.

The selection of the soft-clipped sequencing reads may be performed, for example, on the basis of one or more suitable data files, preferably BAM, SAM or CRAM files. These files may be searched for the presence of soft-clipped regions. In a preferred embodiment, a size cut-off for the size of the soft-clipped region may be implemented. For example, a cut-off of about at least 10 nucleotides may be used. More preferably, a cut-off of at least 11, 12, 13, 14 or 15, or 12 to 15 nucleotides may be used. Particularly preferred is a cut-off of at least 15 nucleotides. Higher cut-off values of 16, 17, 18, 19, 20 and more nucleotides are also envisaged by the present invention. The cutoff values may further be adapted to type, length and form of sequencing reads. In a preferred embodiment, a sequencing read length of 80 nucleotides is used as basis for the calculation of the cut-off values. Moreover, the choice of alignment tools may have an influence on the cut-off value. The cut-off value chosen should, furthermore, be large enough to avoid the accumulation of false positive identification events during subsequent mapping steps.

The searching approach may, for example, be a procedure including the opening of one or more suitable data files, the identification of the presence of soft-clipped nucleotides, the identification of the number of soft-clipped nucleotides, a comparison with a pre-defined cut-off value, e.g. as defined herein above, and the selection of sequencing reads falling within the predefined group for further analysis. The process may either be a single analysis approach, or a continuous or repeated approach, e.g. if sequencing data are stored continuously, or if modifications to the data file(s) are given.

Information on the sequences and the positions of soft-clipped regions may, in specific embodiments, be stored in a suitable data file. This information may further advantageously be stored separately, e.g. in a different file.

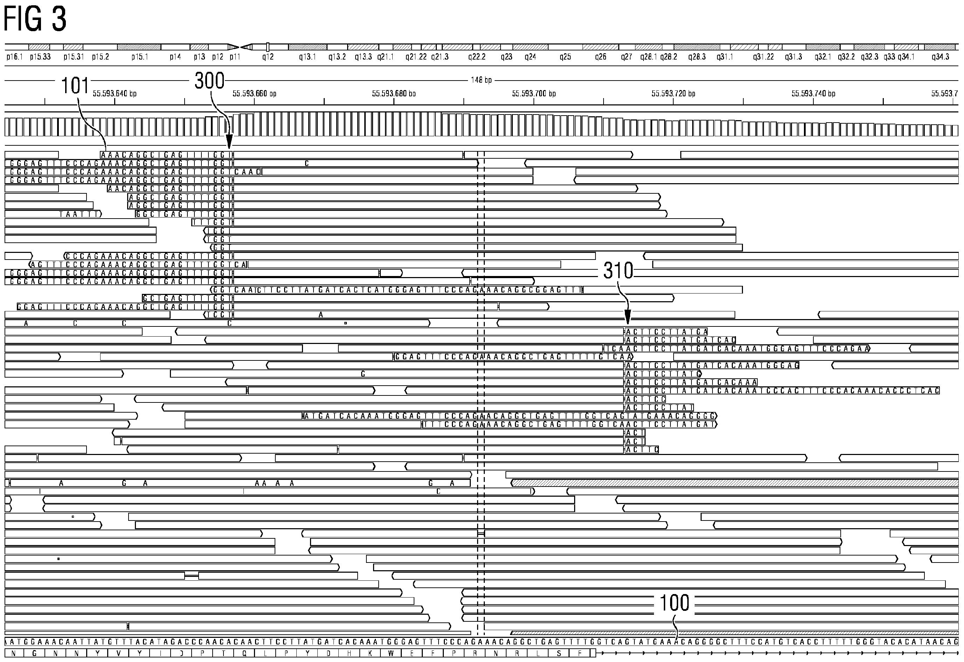

In a next step, the selected soft-clipped sequencing reads are grouped together in accordance with the presence of the partially mapping (i.e. soft-clipped) regions at the start or end portion of the sequencing read. These groups or families of sequencing reads are preferably grouped such that the sequencing reads have an identical start or end position of the soft-clipped-region of the reads. This procedure is, for example, illustrated in Fig. 3, which shows two groups of different soft-clipped (partially mapping) sequencing reads, which have same starting or ending positions of the soft-clipped-regions of the read.

In a specifically preferred embodiment of the present invention, a filtering step is applied, in which the groups or families of sequencing reads are eliminated or discarded from further examination, which have a group of members less than a predefined cut-off value. For example, the families or groups shall have at least 1, 2, 3, 4, 5, 6, 7, 8 or more members. It is preferred that groups or families of less than 2, 3, 4, 5, 6, 7, or 8 sequencing reads are discarded from further analysis. This read-support filtering step is assumed to further reduce the number of false positive identification events during subsequent mapping steps.

Subsequently, in a further step, a synthetic consensus sequence for each group or family of sequencing reads is defined. The term "synthetic consensus sequence" as used herein relates to an artificially designed consensus sequence, which is based on the abundance of identical nucleotides at a certain position. Typically, the most abundant nucleotide at any position of the soft-clipped region of the sequencing reads within one group is used as definition for the identity of nucleotides in the synthetic consensus sequence at the corresponding positions. In case of equivalent abundance at certain positions, all relevant consensus sequence variants may be kept. Alternatively, the consensus sequence may be based on the abundance and quality scores of identical nucleotides at certain positions. In a further embodiment, the most abundant or the most probable nucleotides at any position of the soft-clipped region may be introduced into the consensus sequence.

In a further specifically preferred embodiment of the present invention, a further filtering step is applied, in which only those synthetic consensus sequences are used, which are identical to the sequences of a predefined number of sequencing reads in the group of nucleic acid sequencing reads as defined in above. For example, the corresponding predefined number of sequencing reads may be 1, 2, 3, 4 or more. It is preferred that said number is at least 2, more preferably at least 3, and most preferably at least 4 or more. This consensus-filtering step is assumed to further reduce the number of false positive identification events during subsequent mapping steps. In a specific embodiment, the number of sequencing reads is kept compatible with the minimum number of members in a group of members as defined herein above. The term "compatible" as used herein means that, if a certain cut-off for the group of members is established, e.g. 4, the cut-off for the number of sequencing reads may not be higher, e.g. be 4 or less. Generally, the higher the number of sequencing reads, the stricter the filtering becomes. This may lead to a reduced sensitivity and an increased specificity.

The corresponding cut-offs may thus be adjusted in accordance with required sensitivity and specificity. The skilled person would be enabled to select suitable sensitivity and specificity values, e.g. on the basis of literature sources.

In a further step, reasonable combinations between groups or families of nucleic acid sequencing reads as defined herein above, wherein soft-clipped nucleotides are at the start of the nucleic acid sequence with groups or families of nucleic acid sequencing reads, wherein soft-clipped nucleotides are at the end of the nucleic acid are detected. This may be achieved by a comparison of the synthetic consensus sequences as defined herein above and a reference sequence as defined herein, e.g. a genomic reference sequence or a modified genomic reference sequence as defined herein. Such combinations, i.e. the identification of pairs of synthetic consensus sequences as defined herein, are considered to constitute potential candidates for genomic breakpoints.

In preferred embodiments, this step may provide information on a structural genomic rearrangement, i.e. it may elucidate whether a duplication, a deletion or an inversion is present.

For example, a genomic duplication may be given, if the ending position with respect to the reference sequence of the soft-clipped regions of the groups of nucleic acid sequencing reads which have a partially aligning portion at the start of the mapped nucleic acid sequence is smaller than the starting position with respect to the position in the reference sequence of the soft-clipped regions of said groups of nucleic acid sequencing reads which have a soft-clipped region at the end of the mapped nucleic acid sequence read. For example, a combination of soft-clipped sequencing reads may be considered as candidates for duplication, if the starting positions of the soft-clipped sequencing reads with the soft-clipped region at the start is smaller than the starting positions of soft-clipped sequencing reads with the soft-clipped region at the end, with respect to the position scheme of the reference sequence. The comparison with the reference sequence may in this scenario be a comparison with the sense orientation (plus strand) of said reference sequence.

Similarly, a genomic deletion may be given, if the ending position with respect to the reference sequence of the soft-clipped regions of the groups of nucleic acid sequencing reads which have a partially aligning portion at the start of the mapped nucleic acid sequence is larger than the starting position with respect to the position in the reference sequence of the soft-clipped regions of the groups of nucleic acid sequencing reads which have a soft-clipped region at the end of the mapped nucleic acid sequence. For example, a combination of soft-clipped sequencing reads may be considered as candidates for a deletion, if the starting positions of the soft-clipped sequencing reads with the soft-clipped region at the start is larger than the starting positions of soft-clipped sequencing reads with the soft-clipped region at the end, with respect to the position scheme of the reference sequence. The comparison with the reference sequence may in this scenario be a comparison with the sense orientation (plus strand) of said reference sequence.

As further option, a genomic inversion may be given, if pairs of groups of nucleic acid sequencing reads which comprise a soft-clipped region can be formed, for which both members of the pair have a soft-clipped region at the start of the mapped nucleic acid sequence, or if both members of the pair have a soft-clipped region at the end of the mapped nucleic acid sequence. The comparison with the reference sequence may in this scenario be a comparison with a reverse complementary reference sequence.

In a further specifically preferred embodiment of the present invention, an additional filtering step is applied, in which combinations of nucleic acid sequencing reads are discarded form further analysis, which are characterized by repetitive consensus sequences.

In yet another specifically preferred embodiment of the present invention, an alternative or additional filtering step is applied, in which combinations of nucleic acid sequencing reads as defined above are discarded from further analysis, which show a certain distance between the paired soft-clipped positions of the nucleic acid sequencing reads with respect to the reference sequence. This distance may be, for example, a distance of more than about 100 kb, more than about 75 kb, more than about 50 kb, more than about 45 kb, more than about 40 kb, more than about 35 kb, more than about 30 kb, more than about 25 kb, more than about 20 kb, more than about 15 kb. Most preferably, the distance may be more than about 35 kb, or any suitable value in between the mentioned values.

Subsequently, nucleic acid sequencing reads which match at respective positions of the reference sequence are paired. The reference sequence in case of duplications and deletions is sense oriented reference sequence. In case of inversions, a reverse complementary reference sequence may be employed.

In a final step, a structural genomic rearrangement can be identified, if both synthetic consensus sequences of pairs as mentioned above match at respective positions in the reference sequence. The reference sequence in case of duplications and deletions is a sense oriented reference sequence. In case of inversions, the reference sequence is a reverse complementary reference sequence. Thus, only if both synthetic consensus sequences indeed match at respective positions in the reference sequence, a true structural genomic rearrangement can be assumed to be given. This step allows for a further suitable discarding of a number of false positive candidates. In specific embodiments, the matching may also take place at off-set positions. For example, an off-set of 1 to 15 nucleotides may be used to account for structural genomic rearrangements in repetitive sequences.

In further specific embodiments, additional filtering steps may be applied. For example, the method may comprise a step of elucidating the sequencing depth at the position of the detected structural genomic rearrangement. For example, in a specific embodiment, in case a sequencing depth of about less than 2x, more preferably, less than about 5x, 10x, 20x or 30x at a predefined position of interest, e.g. a known mutational hotspot or known cancer gene, is given, the performance of the method may be stopped. Alternatively, in such a scenario, the performance of the method may not be stopped. The decision on the stopping may further be made dependent on the accordance between the sequencing reads, with a high accordance (e.g. above 95%) or identity speaking for a continuation of the method even in case of low sequencing depth, and a low accordance (e.g. below 95%) speaking against such a continuation.

Furthermore, the method may comprise a step of elucidating the position of the detected structural genomic rearrangement with respect to annotated functional information. Such annotated functional information may comprise, for example, the gene name, location in intron, exon, promoter, enhancer, telomeric, pseudogenic, or repetitive regions. In case the location of the detected structural genomic rearrangement is in a predefined gene, the result may be disregarded, or alternatively, kept. Further, if the location of the detected structural genomic rearrangement is in a predefined intron, the result may be disregarded, or, alternatively, be kept.

Further, if the location of the detected structural genomic rearrangement is in a predefined exon, the result may be disregarded, or, alternatively, be kept. Further, if the location of the detected structural genomic rearrangement is in a predefined enhancer structure, the result may be disregarded, or, alternatively, be kept. Further, if the location of the detected structural genomic rearrangement is in a predefined telomeric region, the result may be disregarded, or, alternatively, be kept. Further, if the location of the detected structural genomic rearrangement is in a predefined pseudogenic region, the result may be disregarded, or, alternatively, be kept. Further, if the location of the detected structural genomic rearrangement is in a predefined repetitive regions, the result may be disregarded, or, alternatively, be kept. Further potential scenarios of structural genomic rearrangement locations include an exonic overlap of duplications or deletions, for example a duplication with break points in two introns surrounding an exon. If such a scenario is identified, the result may be disregarded, or, alternatively, be kept. It is preferred that the result be kept, more preferably be highlighted or tagged.

In a further aspect the present invention relates to an in vitro method to detect structural genomic alterations for stratifying patients for cancer therapy, comprising: performing a massively parallel nucleic acid sequencing of nucleic acids extracted from a patient tumor sample; identifying a structural genomic rearrangement according to the method as defined herein; and attributing the detection of a structural genomic rearrangement to the presence of genomic alterations which can guide a treatment decision.

The term "stratifying patients" as used herein means that patients are partitioned by a factor other than the treatment itself. This factor, may, in the present case, be the presence or absence of a structural genomic rearrangement as defined herein above. The stratification may, for example, help to control confounding variables, or to facilitate the detection and interpretation between variables. Typically, the patient may be analyzed with respect to the presence of structural genomic rearrangement. In case such structural genomic alterations are encountered or suspected, specific therapy forms or specifically adjusted therapy forms may be used.

The term "cancer therapy" as used herein relates to any suitable therapeutic treatment of a cancer disease or a tumor as known to the skilled person. The treatment includes chemotherapy, a treatment with small molecules, an antibody-treatment, or a combination thereof. Also envisaged are additional therapy forms including gene-therapy, antisense-RNA therapy etc. as well as any other suitable type of treatment, including future therapy forms. The skilled person would be aware of the corresponding therapy forms and also the usability of compounds and compositions for specific cancer forms, or can derive this information from suitable literature sources such as Karp and Falchook, Handbook of targeted cancer therapy, 2014. Ed. Lippincott Williams.

The "cancer" form to be treated may be any cancer known to the skilled person, e.g. a cancer form, which can be associated with structural genomic rearrangements, preferably with structural genomic rearrangements as identifiable according to the present invention. This may, for example, be breast cancer, prostate cancer, ovarian cancer, renal cancer, lung cancer, pancreas cancer, urinary bladder cancer, uterus cancer, kidney cancer, brain cancer, stomach cancer, colon cancer, melanoma or fibrosarcoma, gastrointestinal stromal tumor (GIST), glioblastoma and hematological leukemia and lymphomas, both from the myeloid and lymphatic lineage.

The in vitro method according to the present invention, in particular, envisages the performance of a massively parallel nucleic acid sequencing of nucleic acids. It is preferred to carry out this sequencing as described herein above in detail, or as derivable from any suitable literature source. The nucleic acid, e.g. DNA, to be used for the sequencing may be derived from any suitable sample. It is preferred to extract the nucleic acids from a tumor sample of a patient. Also envisaged is to obtain a non-tumorous control sample, or to make use of previously deposited samples, e.g. samples derived from the umbilical cord.

The sample to be used may preferably be a sample comprising one or more premalignant or malignant cells. It may further be a sample comprising cells from a solid tumor or soft-tissue tumor or a metastatic lesion. Also envisaged is the use of a sample comprising tissue or cells from a surgical margin. Further envisaged is the employment of a histologically normal tissue obtained in a biopsy, e.g. as control. The present invention also relates to the use of one or more circulating tumor cells (CTC), e.g. obtained from blood samples. Moreover, the sample may comprise a normal, adjacent tissue (NAT) from a subject having a tumor or being at risk of having a tumor. Additionally, a blood, plasma or serum sample from the same subject having a tumor or being at risk of having a tumor may be used. Further, the sample may be a paraffin or FFPE-sample.

In a particularly preferred embodiment, the in vitro method as mentioned above includes a preparation step for nucleic acids, which comprises a hybrid-capture based nucleic acid enrichment for genomic regions of interest. The term "hybrid-capture based nucleic acid enrichment" as used herein, means that firstly a library of nucleic acids is provided, which is subsequently contacted with a library, either being in solution or being immobilized on a substrate, which comprises a plurality of baits, e.g. oligonucleotide baits complementary to a gene or genomic region of interest to form a hybridization mixture; and subsequently separating a plurality of bait/nucleic acid hybrids from the mixture, e.g. by binding to an entity allowing for separation. This enriched mixture may subsequently be purified or further processed. The identity, amount, concentration, length, form etc. of the baits may be adjusted in accordance with the intended hybridization result. Thereby, a focusing on a gene or region of interest may be achieved, since only those fragments or nucleic acids are capable of hybridizing which show complementarity to the bait sequence. The present invention envisages further variations and future developments of the above mentioned approach. Further details would be known to the skilled person, or can be derived from suitable literature sources such as Mertens et al., 2011, Brief Funct Genomics, , 10(6), 374-386; Frampton et al., 2013, Nature Biotechnology,31(11), 1023-1031; Gnirke et al., 2009, Nature Biotechnology,27(2), 182-189 or from Teer et al, 2010, Genome Res, 20(10), 1420-1431.

The term "gene of interest" or "genomic region of interest" relates to any gene or genomic region, which may be associated with cancer, be relevant for cancer, be involved in the etiology of cancer, or be involved in the development of cancer or being known to be associated with response or resistance to a defined therapy. The gene of interest or genomic region of interest may either be a gene typically associated with somatic mutations / alterations in cancer, or with germ-line mutations associated with cancer. Examples of genes or genomic regions can be found in suitable databases, such as for example, the COSMIC (catalogue of somatic mutations in cancer), which can be accessed at http://cancer.sanger.ac.uk/cosmic, the candidate cancer gene database accessible at http://ccgd-starrlab.oit.umn.edu, or database ClinVar, accessible at https://www.ncbi.nlm.nih.gov/clinvar.

In a further preferred embodiment, the in vitro method as described herein above comprises the additional step of providing a report on the obtained results as to the detection of a genomic rearrangement, its attribution to a cancer state, as well as its use for the guidance of a treatment decision. Such a report may be provided in any suitable manner or form, e.g. as electronic file, as electronic file distributed or accessible over the internet, e.g. provided in cloud or deposited on a server, or web-based, e.g. provided on suitable web-site. Alternatively, the report may be provided in paper form. The report may be provided and thus drafted in a corresponding form, to a patient (including information relevant for the patient), a relative or other person associated with the patient (including information relevant for this person), a caregiver (including information relevant for the caregiver), a physician (including information relevant for the physician), an oncologist (including information relevant for the oncologist), or a hospital or clinic (including information relevant for the institution), or third party payors, insurance companies or government offices (including information relevant for these entities). The report may accordingly be redacted, modified, extended or adjusted to the above specified recipient. For example, information relevant for the oncologist, e.g. as to the exact location of a structural genomic rearrangement, may be omitted in the report for the patient etc.

Among the elements the report may comprise, the present invention envisages one or more of the following:
(i) An output from the method performed, which may include the identification of the structural genomic rearrangement and/or of the corresponding wild-type sequence associated with a tumor of the type of the sample (this information may be relevant for the oncologist, physician, hospital and possibly also insurance companies).
(ii) Information on the role of a genomic alteration or structural genomic rearrangement, or of a corresponding wild-type sequence, in a disease. The corresponding information may also comprise information on prognosis of the disease, on known resistance cases and resistance mechanisms, and/or on potential therapeutic options. Also included may be a conclusion on the most promising treatment, or a potential therapy plan. The corresponding information may be derived from suitable databases, or literature sources, e.g. by a medical professional. These sources may also be provided in the report.
(iii) Further included may be information on the likely effectiveness of a therapeutic option, or the acceptability of a therapeutic option. Moreover, information on the advisability of applying the therapeutic option to a patient having a structural genomic rearrangement identified in the report may be given. The corresponding information may be derived from suitable databases, or literature sources. These sources may also be provided in the report.
(iv) Also included may be information, or a recommendation on the administration of a specific drug or compound, as well as the details on potential administration schemes, administration routes, dosage regimen, treatment regimen etc. This may further be extended to the potential administration of additional drugs, e.g. if this information about a patient is already known, or if a co-administration of drugs is necessary or advisable.
(v) Finally, the report may be confined to specific information, e.g. to specific genes or genomic loci. Other, e.g. predefined genes, genomic loci etc. may be excluded for various reasons, e.g. scientific reasons, reasons connected with treatment options etc. It is preferred that the report is limited to alterations in genes of clinical relevance.

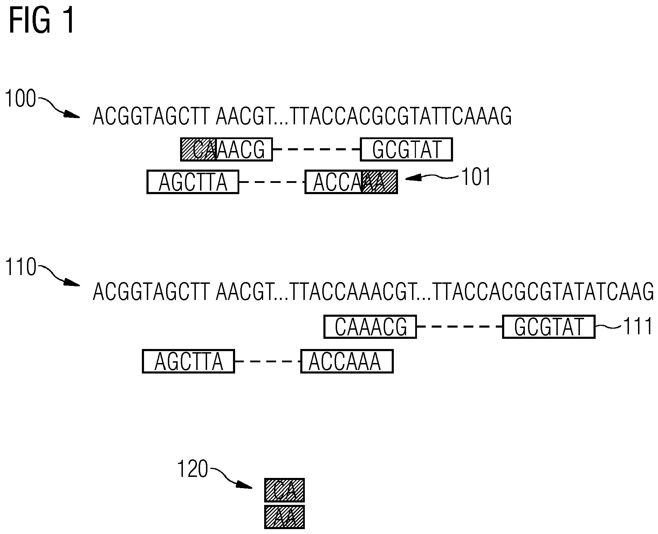

Turning now to FIGURE 1, a reference sequence 100 is shown. Tumor reads (i.e. soft-clipped sequencing reads) 101 are aligned to the reference sequence 100 and display regions, which do not map to the reference. Non-aligning nucleotides are shaded. These nucleotides are ignored in the alignment information, e.g. of a BAM, SAM or CRAM file, but can be derived from the files due to their soft-clipped character. The soft-clipped elements of 101 are additionally shown as 120. Further shown is a tumor genome sequence 110 with corresponding tumor reads (soft-clipped sequencing reads mapped to the tumor genome) 111. The reads 111 correspond to the reads 101 from above, but in this situation no soft-clipping is necessary since complete alignment is possible, as the tumor reads are derived from the tumor genome. The aim of the present invention is to reconstruct the sequence of tumor genome 110 on the basis of the soft-clipped sequencing reads 101.

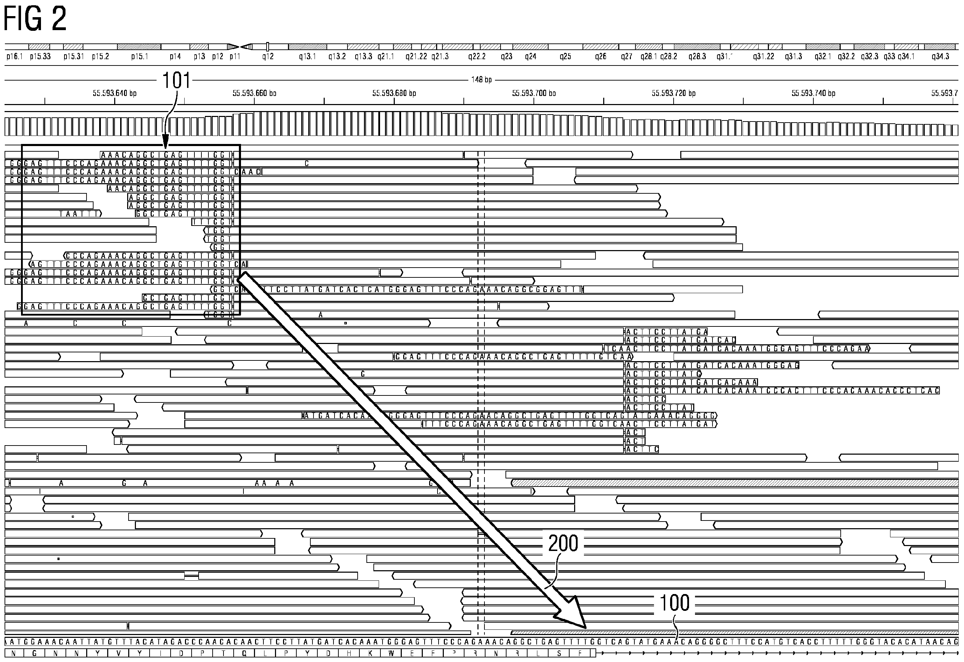

FIGURE 2 illustrates a situation in which a match of a soft-clipped region indicates a structural genomic rearrangement of the duplication-type 200. Accordingly, soft-clipped sequencing reads 101 are grouped and can be matched with reference sequence 100 according to the methodology of the present invention as described herein.

FIGURE 3 shows the start positions of soft-clipped regions 310 and the end positions of soft-clipped regions 300 in a duplication-type structural genomic rearrangement. The definition of these regions is an essential step in the methodology of the present invention as described herein.

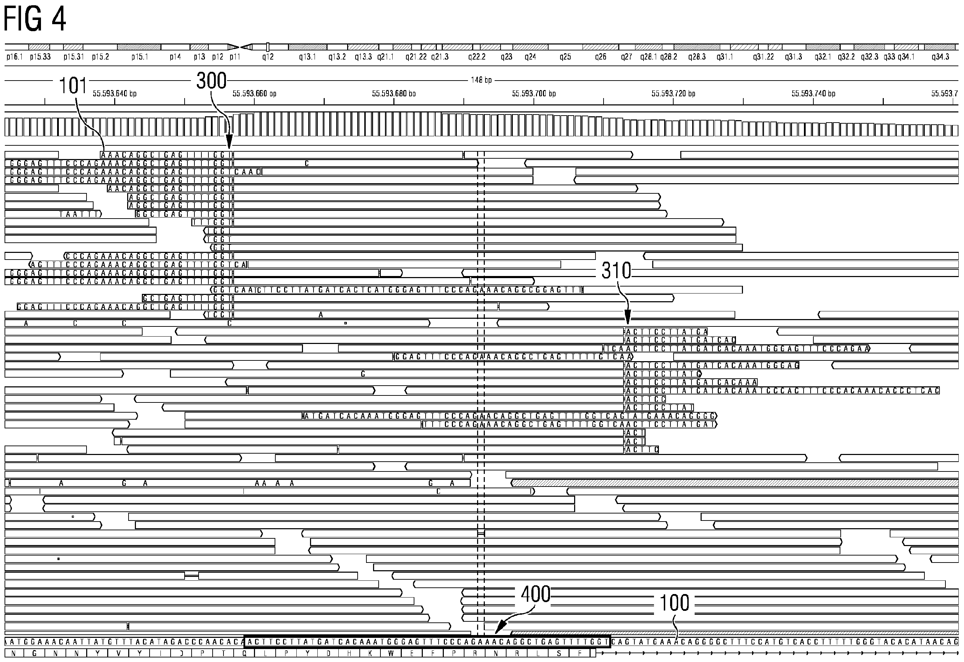

FIGURE 4 depicts the same situation as FIGURE 3 with the start positions 310 of soft-clipped regions 101 and the end positions 300 of soft-clipped regions 101 being indicated. These positions are compared with reference sequence 100. The duplicated sequence 400, as identified after the performance of the methods according to the invention, is shown.

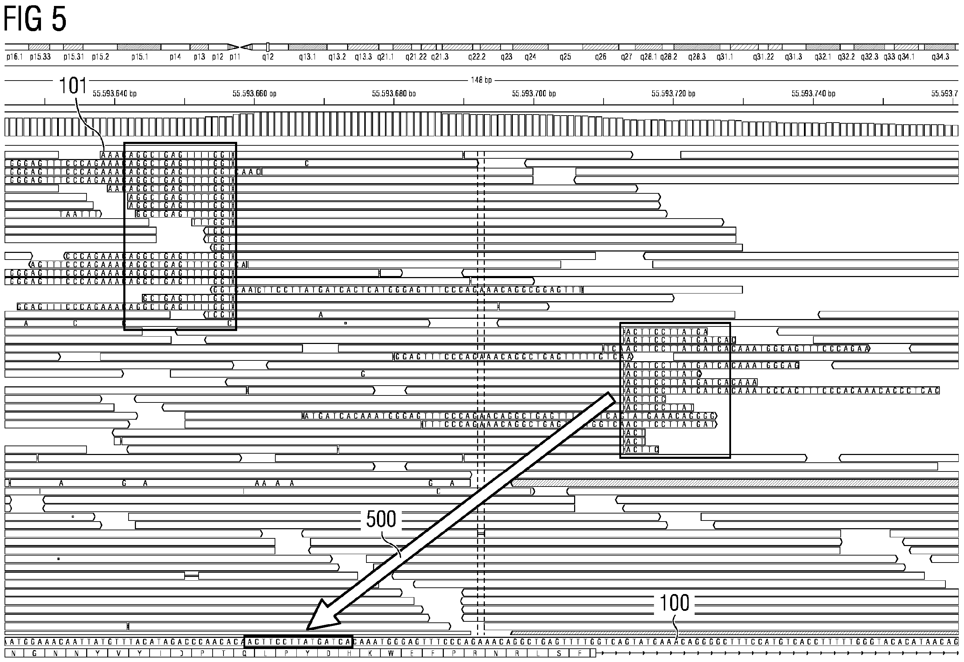

FIGURE 5 shows a situation in which soft-clipped sequences 101 of one genomic breakpoint map to a reference at another genomic breakpoint. This matching of soft-clipped sequencing reads between breakpoints 500 indicates a duplication.

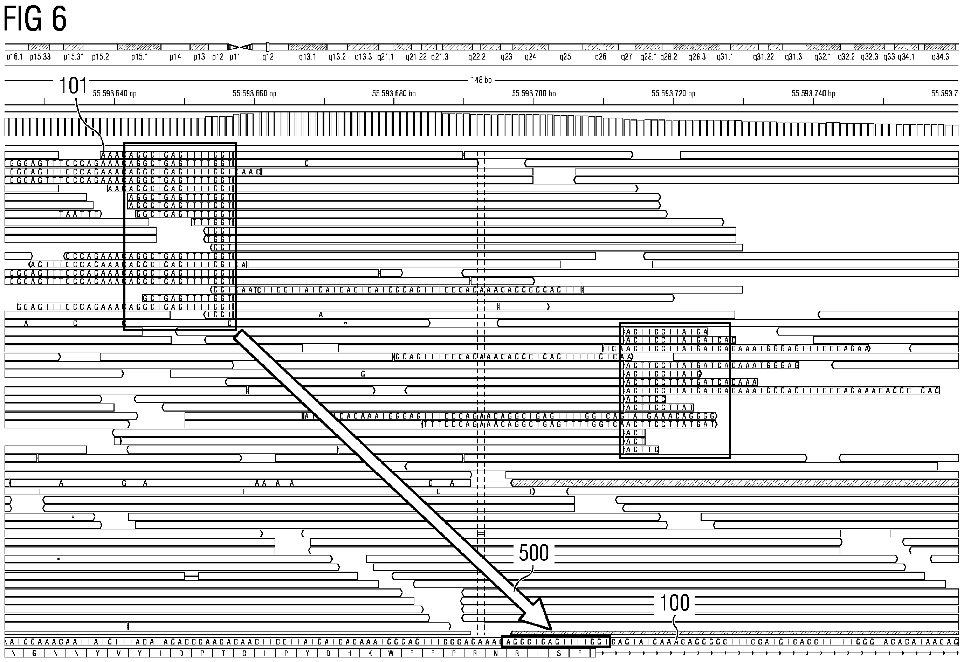

FIGURE 6 depicts the same situation as in Fig. 5, in which soft-clipped sequences 101 of one genomic breakpoint map to a reference at another genomic breakpoint. This matching of soft-clipped sequencing reads between breakpoints 500 also indicates a duplication. Here, the second breakpoint of the duplication is shown. Only if both breakpoints (i.e. the one shown in Fig. 5 and the one shown in Fig. 6) are present, a bona fide duplication has been detected.

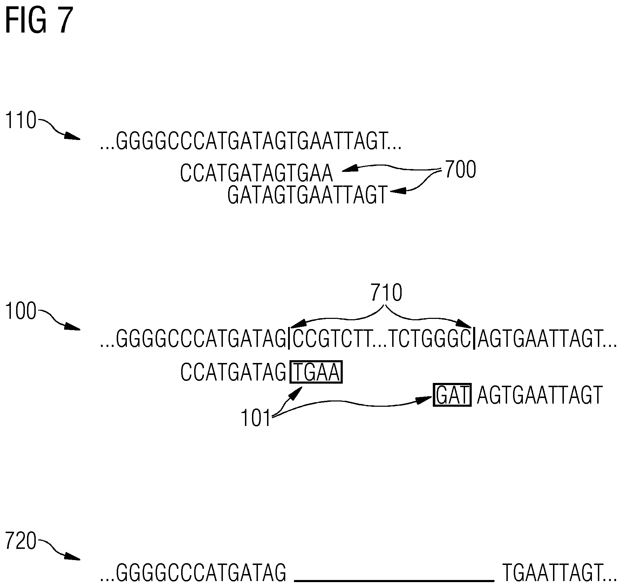

FIGURE 7 shows a tumor genome sequence 110, which matches with a genomic breakpoint spanning sequenced tumor reads 700. When the reads 700 are correspondingly mapped to the reference genome 100, they become partially aligned soft-clipped reads 101. Non-aligning nucleotides are boxed. These nucleotides are ignored in the alignment information, e.g. of a BAM, SAM or CRAM file, but can be derived from the files due to their soft-clipped character. The aim of the present invention is to reconstruct the sequence of tumor genome 110 on the basis of the soft-clipped sequencing reads 101, resulting, for example, in the reconstruction of a deletion situation 720.

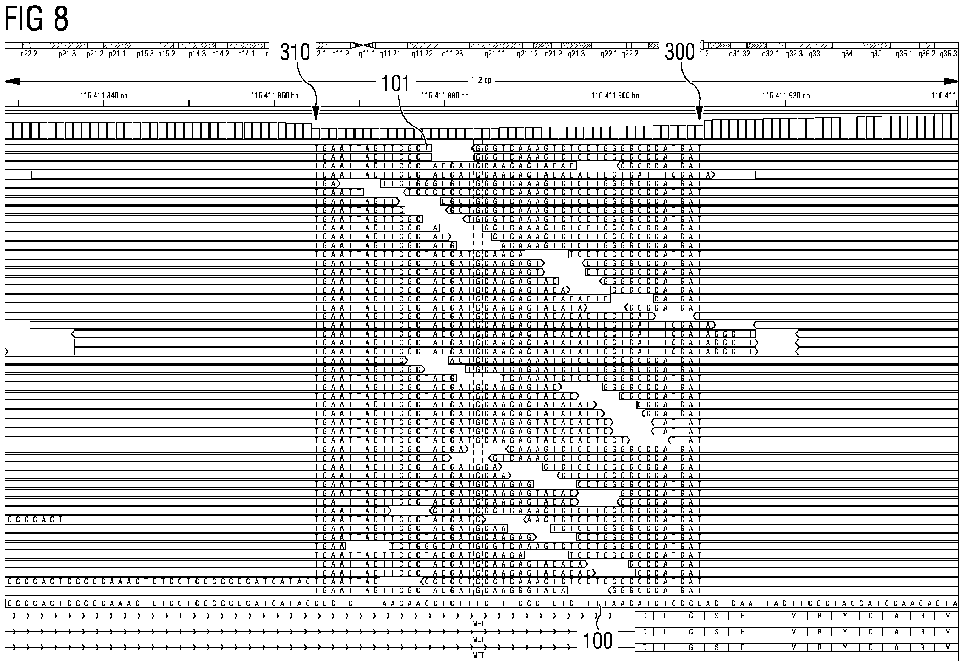

In FIGURE 8 the start positions 310 of the soft-clipped regions of the sequencing reads 101 and the end positions 300 of the soft-clipped regions of the sequencing reads 101 in a deletion-type structural genomic rearrangement are shown. These positions are compared with reference sequence 100.

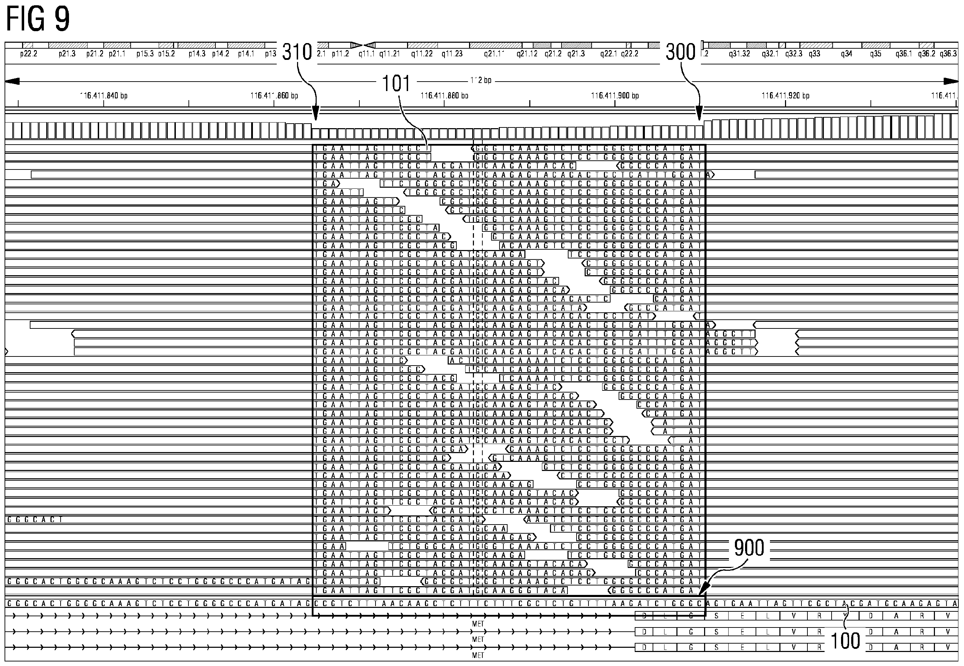

FIGURE 9 shows soft-clipped sequencing reads 101 including the start positions 310 of the soft-clipped regions, and the end positions 300 of the soft-clipped regions. These sequencing reads 101 are only partially mapped to a reference sequence 100 indicating a deletion-type structural genomic rearrangement including deleted sequence portion 900.

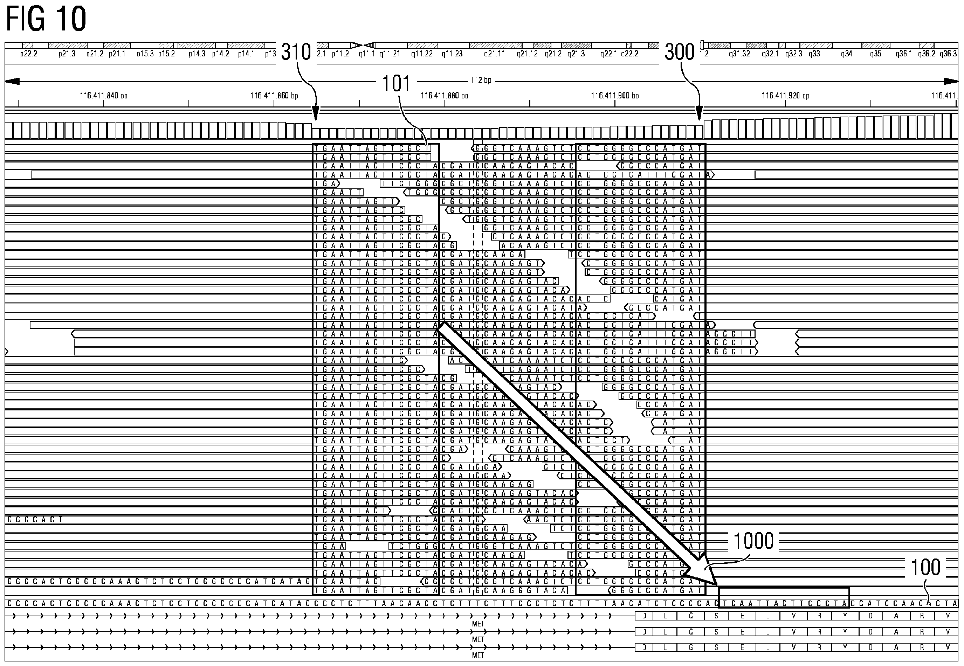

In FIGURE 10 a situation is depicted in which the soft-clipped regions of the sequencing reads 101 including their start positions 310 of one genomic breakpoint map to a reference at another genomic breakpoint, represented by the soft-clipped regions of the sequencing reads 101 and their end positions 300. This matching of soft-clipped sequencing reads between breakpoints 1000 indicates a deletion-type structural genomic rearrangement.

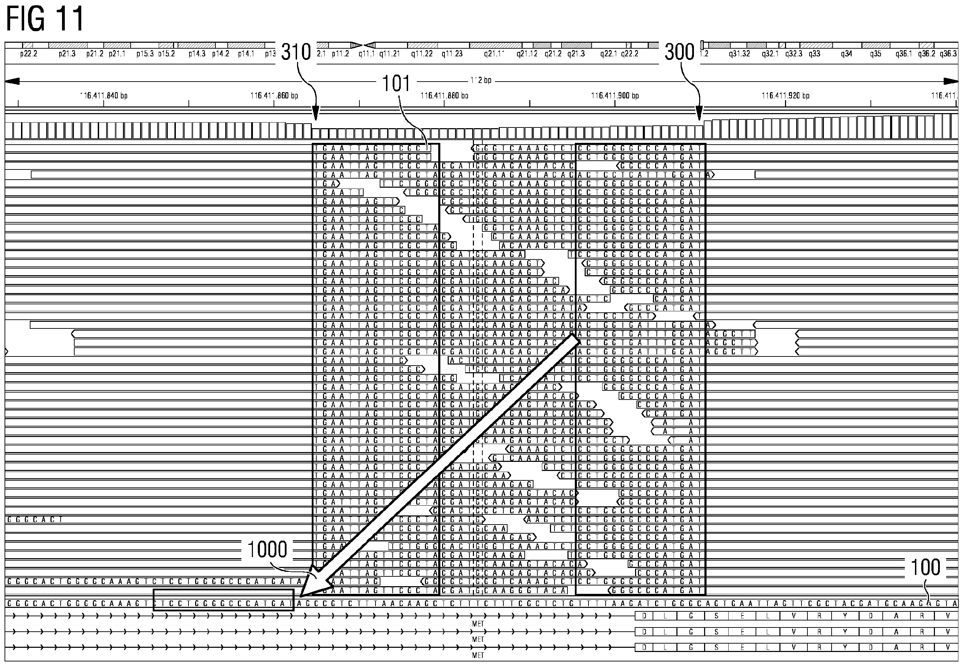

IN FIGURE 11 depicts the same situation as in Fig. 10, in which, however, the soft- clipped regions of the sequencing reads 101 including their end positions 300 of one genomic breakpoint map to a reference at another genomic breakpoint, represented by the soft-clipped regions of the sequencing reads 101 and their start positions 310. This matching of soft-clipped sequencing reads between breakpoints 1000 indicates a deletion-type structural genomic rearrangement. Only if both breakpoints (i.e. the one shown in Fig. 10 and the one shown in Fig. 11) are present, a bona fide deletion has been detected.

### LIST OF REFERENCE NUMERALS

- 100: Reference sequence
- 101: Soft-clipped sequencing read mapped to reference genome
- 110: Tumor genome sequence
- 111: Sequenced tumor reads mapped to tumor genome
- 120: Soft-clipped parts of the reads
- 200: Match of soft-clipped region indicating duplication
- 300: End position of soft-clipped regions
- 310: Start position of soft-clipped regions
- 400: Duplicated sequence
- 500: Matching of soft-clipped sequencing reads between breakpoints indicating duplication
- 700: Sequenced tumor reads
- 710: Genomic breakpoint as inferred from soft-clipped reads
- 720: Reconstruction of deletion situation
- 900: Deleted sequence portion
- 1000: Matching of soft-clipped sequencing reads between breakpoints indicating deletion

## Claims

1. A computer implemented method of identifying a structural genomic rearrangements in massively parallel nucleic acid sequencing data, comprising:
(a) obtaining massively parallel sequencing information for one or more genomic regions as nucleic acid sequence reads;
(b) aligning said nucleic acid sequencing reads to one or more reference sequences;
(c) selecting nucleic acid sequencing reads which only partially map to said reference sequence, wherein a portion of the nucleic acid sequencing reads remains unmapped, constituting a soft-clipped region, wherein the soft-clipped nucleotides of the nucleic sequencing read is at least 8 to 15 nucleotides long;
(d) creating groups of nucleic acid sequencing reads as selected in step (c), all of which are defined by identical start or end positions of said soft-clipped regions;
(e) generating a synthetic consensus sequence for each group as obtained in step (d), wherein said consensus sequence is based on the abundance of identical nucleotides at a position;
**Characterized in that** it further comprises:
(f) identification of pairs of synthetic consensus sequences as candidates for structural genomic rearrangements by generating combinations of positions between (i) groups of nucleic acid sequencing reads, wherein soft-clipped nucleotides are at the start of the nucleic acid sequence, and (ii) groups of nucleic acid sequencing reads, wherein said soft-clipped nucleotides are at the end of the nucleic acid sequence, by comparing the synthetic consensus sequence of step (e) with the reference sequence;
(g) pairing nucleic acid sequencing reads which match at respective positions in the reference sequence; and
(h) detecting a structural genomic rearrangement if both synthetic consensus sequences of pairs as obtained in step (g) match at respective positions in the reference sequence.

2. The method of claim 1, wherein the rearrangement is a deletion, a duplication or an inversion.

3. The method of claim 1 or 2, wherein alignment operations and sequence comparisons are performed with a string matching algorithm.

4. The method of any one of claims 1 to 3, wherein the massively parallel sequence information is provided in a format providing information on alignment and soft-clipped regions, preferably the BAM, SAM or CRAM format.

5. The method of any one of claims 1 to 4, where the nucleic acid sequencing reads have a length of about 50 nucleotides to 50 kb.

6. The method of any one of claims 1 to 5, wherein in the soft-clipped sequencing reads obtained in step (c) information on the position of mapped portion of said reads is stored electronically.

7. The method of any one of claims 1 to 6, wherein in step (d) groups are discarded which comprise less than a predefined number of members.

8. The method of claim 7, wherein said predefined number of members is 1, 2, 3, 4, 5, 6, 7 or 8.

9. The method of any one of claims 1 to 8, wherein the synthetic consensus sequence is identical to a predefined number of sequencing reads in the group of nucleic acid sequencing reads as defined in (d).

10. The method of claim 9, wherein said predefined number of sequencing reads is 1, 2, 3, 4 or more.

11. The method of any one of claims 1 to 10, wherein in step (f) combinations of nucleic acid sequencing reads are discarded form further analysis, which show a distance between the soft-clipped positions of the nucleic acid sequencing reads with respect to the reference sequence of more than 100 kb, preferably more than 35 kb.

12. The method of any one of claims 1 to 11, wherein the method comprises an additional step of elucidating the sequencing depth at the position of the detected structural genomic rearrangement and/or the position of the detected structural genomic rearrangement with respect to annotated functional information, preferably the gene name, or the location in intron, exon, promoter, enhancer, telomeric, pseudogenic, repetitive regions.

13. The method of any one of claims 1 to 12, wherein combinations of positions between groups of nucleic acid sequencing reads as obtained in step (f) are considered to represent:
(i) a duplication, if the ending position with respect to the reference sequence of the soft-clipped regions of said groups of nucleic acid sequencing reads which have a partially aligning portion at the start of the mapped nucleic acid sequence is smaller than the starting position with respect to the reference sequence of the soft-clipped regions of said groups of nucleic acid sequencing reads which have a soft-clipped region at the end of the mapped nucleic acid sequence read;
(ii) a deletion, if the ending position with respect to the reference sequence of the soft-clipped regions of said groups of nucleic acid sequencing reads which have a partially aligning portion at the start of the mapped nucleic acid sequence is larger than the starting position with respect to the reference sequence of the soft-clipped regions of said groups of nucleic acid sequencing reads which have a soft-clipped region at the end of the mapped nucleic acid sequence; or
(iii) an inversion, if pairs of said groups of nucleic acid sequencing reads which have a soft-clipped region can be formed, for which both members of the pair have a soft-clipped region at the start of the mapped nucleic acid sequence, or if both members of the pair have a soft-clipped region at the end of the mapped nucleic acid sequence.

14. An in vitro method to detect structural genomic alterations for stratifying patients for cancer therapy, comprising:
(a) performing a massively parallel nucleic acid sequencing of nucleic acids extracted from a patient tumor sample;
(b) identifying a structural genomic rearrangement according to the method of any one of claims 1 to 13; and
(c) attributing the detection of a genomic rearrangement to the presence of genomic alterations which can guide a treatment decision.

15. The method of claim 14, additionally comprising a preparation step for nucleic acids extracted from a patient sample, which precedes step (a), comprising a hybrid-capture based nucleic acid enrichment for genomic regions of interest.

16. The method of claim 15, wherein said genomic region of interest is a gene or region known to be relevant in cancer.

17. The method of any one of claims 14 to 16, wherein said sample comprises one or more premalignant or malignant cells; cells from a solid tumor or soft-tissue tumor or a metastatic lesion; tissue or cells from a surgical margin; a histologically normal tissue obtained in a biopsy; one or more circulating tumor cells (CTC); a normal, adjacent tissue (NAT) from a subject having a tumor or being at risk of having a tumor; or a blood, plasma or serum sample from the same subject having a tumor or being at risk of having a tumor; or an paraffin or FFPE-sample.

18. The method of any one of claims 14 to 17, wherein said cancer is breast cancer, prostate cancer, ovarian cancer, renal cancer, lung cancer, pancreas cancer, urinary bladder cancer, uterus cancer, kidney cancer, brain cancer, stomach cancer, colon cancer, melanoma or fibrosarcoma, gastrointestinal stromal tumor (GIST), glioblastoma and hematological leukemia and lymphomas, both from the myeloid and lymphatic lineage.

19. The method of any one of claims 14 to 18, wherein the method further comprises providing a report in electronic, web-based, or paper form, to a patient or to another person or entity, a caregiver, a physician, an oncologist, a hospital, clinic, third party payor, insurance company or government office.

20. The method of claim 19, wherein the report comprises one or more of:
(i) output from the method, comprising the identification of the structural genomic rearrangement or wild-type sequence associated with a tumor of the type of the sample;
(ii) information on the role of a genomic alteration, or corresponding wild-type sequence, in a disease, wherein said information comprises information on prognosis, resistance, or potential or suggested therapeutic options, wherein said information is derived from a database, or a literature source;
(iii) information on the likely effectiveness of a therapeutic option, the acceptability of a therapeutic option, or the advisability of applying the therapeutic option to a patient having a structural genomic rearrangement identified in the report, wherein said information is derived from a database, or a literature source;
(iv) information, or a recommendation on the administration of a drug, the administration at a preselected dosage, or in a preselected treatment regimen, in combination with other drugs, to the patient; or
(v) wherein not all structural genomic rearrangements identified in the method are specified in the report, the report can be limited to alterations in genes of clinical relevance.

## Patentansprüche

1. Computer-implementiertes Verfahren zum Identifizieren struktureller genomischer Umlagerungen in Daten von massiver Nukleinsäure-Parallelsequenzierung, umfassend:
(a) Erhalten von Informationen aus massiver Parallelsequenzierung für eine oder mehrere Genomregionen als Nukleinsäuresequenzreads;
(b) Ausrichten der Nukleinsäuresequenzierungsreads mit einer oder mehreren Referenzsequenzen;
(c) Auswählen der Nukleinsäuresequenzierungsreads, die nur partiell auf die Referenzsequenz kartieren, wobei ein Abschnitt der Nukleinsäuresequenzierungsreads unkartiert bleibt und eine Soft-Clipped-Region bildet, wobei die Soft-Clipped-Nukleotide des Nukleinsäuresequenzierungsreads mindestens 8 bis 15 Nukleotide lang sind;
(d) Erstellen von Gruppen von Nukleinsäuresequenzierungsreads, wie in Schritt (c) ausgewählt, die sämtlich durch identische Anfangs- oder Endpositionen der Soft-Clipped-Regionen definiert sind;
(e) Erzeugen einer synthetischen Konsensussequenz für jede Gruppe, wie in Schritt (d) erhalten, wobei die Konsensussequenz auf der Häufigkeit identischer Nukleotide an einer Position basiert;
**dadurch gekennzeichnet, dass** es ferner umfasst:
(f) Identifikation von Paaren von synthetischen Konsensussequenzen als Kandidaten für strukturelle genomische Umlagerungen durch Erzeugen von Kombinationen von Positionen zwischen (i) Gruppen von Nukleinsäuresequenzierungsreads, wobei sich Soft-Clipped-Nukleotide am Anfang der Nukleinsäuresequenz befinden, und (ii) Gruppen von Nukleinsäuresequenzierungsreads, wobei sich die Soft-Clipped-Nukleotide am Ende der Nukleinsäuresequenz befinden, durch Vergleichen der synthetischen Konsensussequenz von Schritt (e) mit der Referenzsequenz;
(g) Paaren von Nukleinsäuresequenzierungsreads, die an den jeweiligen Positionen in der Referenzsequenz übereinstimmen und
(h) Nachweisen einer strukturellen genomischen Umlagerung, wenn beide synthetischen Konsensussequenzen von Paaren, wie in Schritt (g) erhalten, an entsprechenden Positionen in der Referenzsequenz übereinstimmen.

2. Verfahren nach Anspruch 1, wobei es sich bei der Umlagerung um eine Deletion, eine Duplikation oder eine Inversion handelt.

3. Verfahren nach Anspruch 1 oder 2, wobei Ausrichtungsvorgänge und Sequenzvergleiche mit einem String-Matching-Algorithmus durchgeführt werden.

4. Verfahren nach einem der Ansprüche 1 bis 3, wobei die massive parallele Sequenzinformation in einem Format, das Informationen zur Ausrichtung und zu Soft-Clipped-Regionen liefert, vorzugsweise im BAM-, SAM- oder CRAM-Format bereitgestellt wird.

5. Verfahren nach einem der Ansprüche 1 bis 4, wobei die Nukleinsäuresequenzierungsreads eine Länge von etwa 50 Nukleotiden bis 50 kb haben.

6. Verfahren nach einem der Ansprüche 1 bis 5, wobei in den in Schritt (c) erhaltenen Soft-Clipped-Sequenzierungsreads Informationen zur Position des kartierten Abschnitts der Reads elektronisch gespeichert sind.

7. Verfahren nach einem der Ansprüche 1 bis 6, wobei in Schritt (d) Gruppen aussortiert werden, die weniger als eine zuvor festgelegte Anzahl an Mitgliedern umfassen.

8. Verfahren nach Anspruch 7, wobei die zuvor festgelegte Anzahl an Mitgliedern 1, 2, 3, 4, 5, 6, 7 oder 8 beträgt.

9. Verfahren nach einem der Ansprüche 1 bis 8, wobei die synthetische Konsensussequenz mit einer zuvor festgelegten Anzahl an Sequenzierungsreads in der Gruppe von Nukleinsäuresequenzierungsreads, wie in (d) definiert, identisch ist.

10. Verfahren nach Anspruch 9, wobei die zuvor festgelegte Anzahl an Sequenzierungsreads 1, 2, 3, 4 oder mehr beträgt.

11. Verfahren nach einem der Ansprüche 1 bis 10, wobei in Schritt (f) Kombinationen von Nukleinsäuresequenzierungsreads aus der weiteren Analyse aussortiert werden, die einen Abstand zwischen den Soft-Clipped-Positionen der Nukleinsäuresequenzierungsreads in Bezug auf die Referenzsequenz von mehr als 100 kb, vorzugsweise mehr als 35 kb, aufweisen.

12. Verfahren nach einem der Ansprüche 1 bis 11, wobei das Verfahren einen zusätzlichen Schritt der Aufklärung der Sequenzierungstiefe an der Position der nachgewiesenen strukturellen genomischen Umlagerung und/oder der Position der nachgewiesenen strukturellen genomischen Umlagerung in Bezug auf annotierte funktionelle Informationen, vorzugsweise die Genbezeichnung oder die Stelle in Intron-, Exon-, Promotor-, Enhancer-, Telomer-, Pseudogen- und repetitiven Regionen, umfasst.

13. Verfahren nach einem der Ansprüche 1 bis 12, wobei von Kombinationen von Positionen zwischen Gruppen von Nukleinsäuresequenzierungsreads, wie in Schritt (f) erhalten, angenommen wird, dass sie Folgendes darstellen:
(i) eine Duplikation, wenn die Endposition in Bezug auf die Referenzsequenz der Soft-Clipped-Regionen der Gruppen von Nukleinsäuresequenzierungsreads, die einen partiell ausrichtbaren Abschnitt am Anfang der kartierten Nukleinsäuresequenz aufweisen, kleiner ist als die Anfangsposition in Bezug auf die Referenzsequenz der Soft-Clipped-Regionen der Gruppen von Nukleinsäuresequenzierungsreads, die eine Soft-Clipped-Region am Ende des kartierten Nukleinsäuresequenzreads aufweisen;
(ii) eine Deletion, wenn die Endposition in Bezug auf die Referenzsequenz der Soft-Clipped-Regionen der Gruppen von Nukleinsäuresequenzierungsreads, die einen partiell ausrichtbaren Abschnitt am Anfang der kartierten Nukleinsäuresequenz aufweisen, größer ist als die Anfangsposition in Bezug auf die Referenzsequenz der Soft-Clipped-Regionen der Gruppen von Nukleinsäuresequenzierungsreads, die eine Soft-Clipped-Region am Ende der kartierten Nukleinsäuresequenz aufweisen; oder
(iii) eine Inversion, wenn Paare der Gruppen von Nukleinsäuresequenzierungsreads, die eine Soft-Clipped-Region aufweisen, gebildet werden können, bei denen beide Mitglieder des Paars eine Soft-Clipped-Region am Anfang der kartierten Nukleinsäuresequenz haben oder wenn beide Mitglieder des Paares eine Soft-Clipped-Region am Ende der kartierten Nukleinsäuresequenz haben.

14. In-vitro-Verfahren zum Nachweis struktureller genomischer Veränderungen zur Stratifizierung von Patienten für eine Krebstherapie, umfassend:
(a) Durchführen einer massiven parallelen Nukleinsäuresequenzierung von aus einer Tumorprobe eines Patienten extrahierten Nukleinsäuren;
(b) Identifizieren einer strukturellen genomischen Umlagerung gemäß dem Verfahren nach einem der Ansprüche 1 bis 13 und
(c) Zurückführen des Nachweises einer genomischen Umlagerung auf das Vorhandensein genomischer Veränderungen als Entscheidungsleitfaden für eine Behandlung.

15. Verfahren nach Anspruch 14, ferner umfassend einen Vorbereitungsschritt für aus einer Patientenprobe extrahierte Nukleinsäuren, der Schritt (a) vorausgeht und eine auf Hybrid-Capture basierende Nukleinsäureanreicherung von interessierenden Genomregionen umfasst.

16. Verfahren nach Anspruch 15, wobei die interessierende Genomregion ein Gen oder eine Region ist, von dem bzw. der bekannt ist, dass es bzw. sie bei Krebs relevant ist.

17. Verfahren nach einem der Ansprüche 14 bis 16, wobei die Probe eine oder mehrere prämaligne oder maligne Zellen; Zellen aus einem soliden Tumor oder Weichgewebetumor oder einer metastatischen Läsion; Gewebe oder Zellen aus einem Resektionsrand; ein in einer Biopsie gewonnenes histologisch normales Gewebe; eine oder mehrere zirkulierende Tumorzellen (CTC); ein normales, angrenzendes Gewebe (NAT) von einem Individuum mit einem Tumor hat oder mit einem Risiko für einen Tumor; oder eine Blut-, Plasma- oder Serumprobe von demselben Individuum mit einem Tumor oder mit einem Risiko für einen Tumor; oder eine Paraffin- oder FFPE-Probe umfasst.

18. Verfahren nach einem der Ansprüche 14 bis 17, wobei es sich bei dem Krebs um Brustkrebs, Prostatakrebs, Eierstockkrebs, Nierenkrebs, Lungenkrebs, Bauchspeicheldrüsenkrebs, Harnblasenkrebs, Gebärmutterkrebs, Nierenkrebs, Gehirnkrebs, Magenkrebs, Kolonkrebs, Melanom oder Fibrosarkom, gastrointestinalen Stromatumor (GIST), Glioblastom und hämatologische Leukämie und Lymphome, sowohl aus der myeloischen als auch der lymphatischen Abstammungslinie, handelt.

19. Verfahren nach einem der Ansprüche 14 bis 18, wobei das Verfahren ferner das Bereitstellen eines Berichts in elektronischer, Internet-basierter oder Papierform an einen Patienten oder eine andere Person oder juristische Person, eine Pflegekraft, einen Arzt, einen Onkologen, ein Krankenhaus, eine Klinik, einen Drittzahler, eine Versicherungsgesellschaft oder eine Regierungsstelle umfasst.

20. Verfahren nach Anspruch 19, wobei der Bericht eines oder mehrere der Folgenden umfasst:
(i) Ergebnis des Verfahrens, umfassend die Identifikation der strukturellen genomischen Umlagerung oder der Wildtyp-Sequenz in Verbindung mit einem Tumor des Typs der Probe;
(ii) Information hinsichtlich der Rolle einer genomischen Veränderung oder der entsprechenden Wildtyp-Sequenz bei einer Krankheit, wobei diese Information Informationen hinsichtlich der Prognose, Resistenz oder potenzieller oder vorgeschlagener Therapieoptionen umfasst, wobei die Information aus einer Datenbank oder einer Literaturquelle stammt;
(iii) Information hinsichtlich der wahrscheinlichen Wirksamkeit einer Therapieoption, der Angemessenheit einer Therapieoption oder der Ratsamkeit der Anwendung der Therapieoption bei einem Patienten mit einer in dem Bericht genannten strukturellen genomischen Umlagerung, wobei die Information aus einer Datenbank oder einer Literaturquelle stammt;
(iv) Information oder eine Empfehlung hinsichtlich der Verabreichung eines Arzneistoffs, der Verabreichung in einer zuvor gewählten Dosierung oder in einem zuvor gewählten Behandlungsschema, in Kombination mit anderen Arzneistoffen, an den Patienten oder,
(v) wobei nicht alle in dem Verfahren identifizierten strukturellen genomischen Umlagerungen im Bericht genau aufgeführt sind, kann der Bericht auf Veränderungen in Genen von klinischer Relevanz beschränkt sein.

## Revendications

1. Procédé mis en œuvre par ordinateur d'identification de réarrangements génomiques structuraux dans des données de séquençage d'acide nucléique parallèle de manière massive, comprenant :
(a) obtenir une information de séquençage parallèle de manière massive pour une ou pour plusieurs régions génomiques comme lectures de séquence d'acide nucléique ;
(b) aligner lesdites lectures de séquençage d'acide nucléique sur une ou sur plusieurs séquences de référence ;
(c) sélectionner des lectures de séquençage d'acide nucléique, qui correspondent seulement en partie à ladite séquence de référence, dans lequel une partie des lectures de séquence d'acide nucléique reste non cartographiée, en constituant une région soft-clipped, dans lequel les nucléotides soft-clipped de la lecture de séquençage nucléique est d'une longueur d'au moins 8 à 15 nucléotides ;
(d) créer des groupes de lectures de séquençage d'acide nucléique comme sélectionnés au stade (c), tous définis par des positions identiques de début ou de fin desdites régions soft-clipped ;
(e) créer une séquence consensus synthétique pour chaque groupe, tel qu'obtenu au stade (d), dans lequel la séquence consensus repose sur l'abondance de nucléotides identiques en une position ;
**caractérisé en ce qu'**il comprend en outre :
(f) l'identification de paires de séquences consensus synthétiques comme candidats pour des réarrangements génomiques structuraux en créant des combinaisons de positions entre (i) des groupes de lectures de séquençage d'acide nucléique, dans lesquels des nucléotides soft-clipped sont au début de la séquence d'acide nucléique et (ii) des groupes de séquençage d'acide nucléique, dans lesquels les nucléotides soft-clipped sont à la fin de la séquence d'acide nucléique, en comparant la séquence consensus synthétique du stade (e) à la séquence de référence ;
(g) appariement de lectures de séquençage d'acide nucléique, qui correspondent à des positions respectives dans la séquence de référence ; et
(h) détection d'un réarrangement génomique structural, si les deux séquences consensus synthétiques de paires, telles qu'obtenues au stade (g), correspondent à des positions respectives dans la séquence de référence.

2. Procédé suivant la revendication 1, dans lequel le réarrangement est une suppression, une duplication ou une inversion.

3. Procédé suivant la revendication 1 ou 2, dans lequel on effectue les opérations d'alignement et de comparaison de séquences avec un algorithme de correspondance de chaîne.

4. Procédé suivant l'une quelconque des revendications 1 à 3, dans lequel on fournit l'information de séquence parallèle de manière massive dans un format donnant de l'information sur l'alignement et les régions soft-clipped, de préférence le format BAM, SAM ou CRAM.

5. Procédé suivant l'une quelconque des revendications 1 à 4, dans lequel les lectures de séquençage d'acide nucléique ont une longueur d'environ 50 nucléotides à 50 kb.

6. Procédé suivant l'une quelconque des revendications 1 à 5, dans lequel, dans les lectures de séquençage soft-clipped obtenues au stade (c), l'information sur la position de la partie correspondante desdites lectures est mise en mémoire électroniquement.

7. Procédé suivant l'une quelconque des revendications 1 à 6, dans lequel, dans le stade (d), on écarte des groupes, qui comprennent moins qu'un nombre défini à l'avance d'éléments.

8. Procédé suivant la revendication 7, dans lequel le nombre défini d'éléments est 1, 2, 3, 4, 5, 6, 7 ou 8.

9. Procédé suivant l'une quelconque des revendications 1 à 8, dans lequel la séquence consensus synthétique est identique à un nombre défini à l'avance de lectures de séquençage dans le groupe de lectures de séquençage d'acide nucléique, tel que défini dans (d).

10. Procédé suivant la revendication 9, dans lequel le nombre défini à l'avance de lectures de séquençage est 1, 2, 3, 4 ou plus.

11. Procédé suivant l'une quelconque des revendications 1 à 10, dans lequel, dans le stade (f), on écarte des combinaisons de lectures de séquençage d'acide nucléique d'une analyse plus poussée, qui présentent une distance entre les positions soft-clipped des lectures de séquençage d'acide nucléique par rapport à la séquence de référence de plus de 100 kb, de préférence de plus de 35 kb.

12. Procédé suivant l'une quelconque des revendications 1 à 11, dans lequel le procédé comprend un stade supplémentaire d'élucidation de la profondeur de séquençage en la position du réarrangement génomique structural détecté et/ou en la position du réarrangement génomique structural détecté par rapport à une information fonctionnelle annotée, de préférence le nom du gène ou l'emplacement dans des régions d'intron, d'exon, de promoteur, d'activateur, télomères, pseudogéniques, répétitives.

13. Procédé suivant l'une quelconque des revendications 1 à 12, dans lequel on considère que des combinaisons de positions entre des groupes de lectures de séquençage d'acide nucléique, tels qu'obtenus au stade (f), représentent :
(i) une duplication, si la position de fin, par rapport à la séquence de référence des régions soft-clipped desdits groupes de lectures de séquençage d'acide nucléique, qui ont une partie s'alignant partiellement au début de la séquence d'acide nucléique cartographiée, est plus petite que la position de début par rapport à la séquence de régions soft-clipped desdits groupes de lectures de séquençage d'acide nucléique, qui ont une région soft-clipped à la fin de la lecture de séquence d'acide nucléique cartographiée ;
(ii) une suppression, si la position de fin par rapport à la séquence de référence des régions soft-clipped desdits groupes de lectures de séquençage d'acide nucléique, qui ont une partie s'alignant partiellement au début de la séquence d'acide nucléique cartographiée, est plus grande que la position de début par rapport à la séquence des régions soft-clipped desdits groupes de lectures de séquençage d'acide nucléique, qui ont une région soft-clipped à la fin de la séquence d'acide nucléique cartographiée ; ou
(iii) une inversion, si des paires desdits groupes de lectures de séquençage d'acide nucléique, qui ont une région soft-clipped, peut être formée pour lesquelles les deux membres de la paire ont une région soft-clipped au début de la séquence d'acide nucléique cartographiée ou si les deux membres de la paire ont un région soft-clipped à la fin de la séquence d'acide nucléique cartographiée.

14. Procédé in vitro de détection d'altérations génomiques structurales pour classer des patients en thérapie du cancer, comprenant :
(a) on effectue un séquençage d'acide nucléique parallèle d'une manière massive d'acides nucléiques extrait d'un échantillon tumoral du patient ;
(b) on identifie un réarrangement génomique structural suivant le procédé suivant l'une quelconque des revendications 1 à 13 ; et
(c) on attribue la détection d'un réarrangement génomique à la présence d'altérations génomiques, qui peuvent guider une décision de traitement.

15. Procédé suivant la revendication 14, comprenant en outre un stade de préparation d'acides nucléiques extraits d'un échantillon du patient, qui précède le stade (a), comprenant une capture hybride, reposant sur un enrichissement en acide nucléique de régions génomiques, auxquelles on s'intéresse.

16. Procédé suivant la revendication 15, dans lequel la région génomique à laquelle on s'intéresse est un gène ou une région connue pour être pertinente dans le cancer.

17. Procédé suivant l'une quelconque des revendications 14 à 16, dans lequel ledit échantillon comprend une ou plusieurs cellules prémalignes ou malignes ; des cellules d'une tumeur solide ou d'une tumeur de parties molles ou une lésion métastatique ; des tissus de cellules d'une marge chirurgicale ; un tissu normal histologiquement obtenu dans une biopsie ; une ou plusieurs cellules tumorales circulantes (CTC) ; du tissu de voisinage (NAT) d'un sujet ayant une tumeur ou étant en danger d'avoir une tumeur ; ou un échantillon de sang, de plasma ou de sérum du même sujet ayant une tumeur ou en étant en danger d'avoir une tumeur ; ou un échantillon de paraffine ou de FFPE.

18. Procédé suivant l'une quelconque des revendications 14 à 17, dans lequel le cancer est un cancer du sein, un cancer de la prostate, un cancer des ovaires, un cancer rénal, un cancer des poumons, un cancer du pancréas, un cancer de la vessie urinaire, un cancer de l'utérus, un cancer du rein, un cancer du cerveau, un cancer de l'estomac, un cancer du colon, un mélanome ou un fibrosarcome, une tumeur stromale gastrointestinale (GIST), un glioblastome et une leucémie hématologique et des lymphomes, tous deux de la famille des myéloïdes et lymphatiques.

19. Procédé suivant l'une quelconque des revendications 14 à 18, dans lequel le procédé comprend en outre fournir un rapport sous forme électronique sur la toile ou sous forme papier à un patient ou une autre personne ou entité, à un donneur de soin, à un médecin, à un oncologiste, à un hôpital, à une clinique, un payeur tiers, une compagnie d'assurance ou un bureau du gouvernement.

20. Procédé suivant la revendication 19, dans lequel le rapport comprend l'un ou plusieurs de :
(i) ce qui sort du procédé comprenant l'identification du réarrangement génomique structural ou une séquence de phénotype sauvage associée à une tumeur du type de l'échantillon ;
(ii) d'une information sur le rôle d'une altération génomique ou une séquence correspondante de génotype sauvage dans une maladie, dans lequel l'information comprend une information sur le pronostic, la résistance ou des options thérapeutiques potentielles ou proposées, dans lequel ladite information est déduite d'une base de données ou d'une source de littérature ;
(iii) des informations sur l'effectivité probable d'une option thérapeutique, l'acceptabilité d'une option thérapeutique ou qui peut être de donner avis d'appliquer l'option thérapeutique à un patient ayant un réarrangement structural identifié dans le rapport, dans lequel ladite information est déduite d'une base de données ou d'une source de littérature ;
(iv) une information ou une recommandation sur l'administration d'un médicament, l'administration d'un dosage sélectionné à l'avance, ou d'un régime de traitement sélectionné à l'avance, en combinaison avec d'autres médicaments au patient ; ou
(v) dans lequel tous les réarrangements génomiques structuraux identifiés dans le procédé ne sont pas précisés dans le rapport, le rapport pouvant être limité à des altérations de gènes pertinentes du point de vue clinique.
